# EUROPÄISCHE PATENTSCHRIFT

(11) **EP 2 716 139 B1**
(45) Veröffentlichungstag und Bekanntmachung des Hinweises auf die Patenterteilung: **19.07.2017**
(21) Anmeldenummer: 12731316.1
(22) Anmeldetag: 29.05.2012
(51) Int. Cl.: H05H 1/24

(54) **VORRICHTUNG UND VERFAHREN ZUR ERZEUGUNG EINES KALTEN, HOMOGENEN PLASMAS UNTER ATMOSPHÄRENDRUCKBEDINGUNGEN**
DEVICE AND METHOD FOR PRODUCING A COLD, HOMOGENEOUS PLASMA UNDER ATMOSPHERIC PRESSURE CONDITIONS
DISPOSITIF ET PROCÉDÉ DE PRODUCTION D'UN PLASMA FROID HOMOGÈNE DANS DES CONDITIONS DE PRESSION ATMOSPHÉRIQUE

(30) Priorität: 31.05.2011 DE 102011076806
(43) Veröffentlichungstag der Anmeldung: 09.04.2014
(73) Patentinhaber: Leibniz-Institut für Plasmaforschung und Technologie e.V., 17489 Greifswald (DE)
(72) Erfinder: SCHÄFER, Jan, 17491 Greifswald (DE); HORN, Stefan, 17509 Loissin (DE); BRANDENBURG, Ronny, 17495 Gross-Kiesow OT Kessin (DE); FOEST, Ruediger, 17498 Neuenkirchen (DE); STIEBER, Manfred, 17489 Greifswald (DE); WELTMANN, Klaus-Dieter, 18609 Ostseebad Binz (DE)
(74) Vertreter: Junghans, Claas
(86) Internationale Anmeldenummer: PCT/EP2012/059939
(87) Internationale Veröffentlichungsnummer: WO 2012/163876

(56) Entgegenhaltungen:
- WO-A1-2005/076673
- WO-A1-2009/036579
- WO-A1-2010/082561

## Beschreibung

Die Erfindung betrifft eine als Plasma-Intractor (PI) bezeichnete Plasmaquelle zur Erzeugung eines kalten, homogenen Plasmas unter Atmosphärendruckbedingungen entsprechend dem Oberbegriff des Anspruchs 1, die vorteilhaft zur Anregung und Steuerung reaktiver Prozesse in strömenden Medien eingesetzt werden kann.
Unter dem Begriff "kaltes" Plasma wird ein nichtthermisches Niedertemperaturplasma verstanden, das durch folgende Merkmale gekennzeichnet ist:
(i) die Neutralgastemperatur des Plasmas ist vergleichbar oder nur leicht größer als die Umgebungstemperatur (d.h. Labortemperatur, bzw. Temperatur der Vorrichtung oder Temperatur des Arbeitsgases vor der Zündung des Plasmas) und
(ii) die Vorrichtung kann das Plasma ohne jedes aktive Kühlungssystem kontinuierlich über mehrere Stunden erzeugen.

Unter dem Begriff "homogenes" Plasma wird hier eine durch die Vorrichtung erzeugte und über die Spannungsperiode gemittelte Struktur der Entladung verstanden, welche besonders in der zur Elektrodenanordnung bezogenen axialen Richtung eine überraschend kleine Fluktuation der Strahlungsintensität im sichtbaren Spektralbereich aufweist. Diese Eigenschaft stellt einen prinzipiellen Vorteil gegenüber der filamentierten Struktur geläufiger dielektrisch behinderter Entladungen unter Atmosphärendruck dar.

### Stand der Technik

In der Fach- bzw. Patentliteratur werden zahlreiche Plasmaquellen und plasmatechnologische Verfahren zur Reinigung oder Aktivierung von Oberflächen unter Atmosphärendruckbedingungen beschriebenen (Review-Artikel: K. H. Becker et al., J. Phys. D: Appl. Phys. 39 (2006), R55; M. Laroussi at al, Plasma Process. Polym. 4(2007), 777 sowie F. Iza, et al., Plasma Process. Polym. 5 (2008), 322). Bei Einspeisung eines als Schichtbildner (Präkursor) geeigneten Mediums zusätzlich zum Prozessgas in die Plasmazone können Plasmaquellen dieser Art auch zur Plasmabeschichtung von Oberflächen mit dem Ziel verwendet werden, die Oberflächeneigenschaften eines Materials in Bezug auf Chemikalienresistenz, Benetzbarkeit, Haftung, Kratzfestigkeit, Gasdurchlässigkeit, tribologische, optische, dielektrische Parameter, etc. dauerhaft zu verbessern (Beispiele: J. Janča et al., Surf. Coat. Technol. 547 (1999), 116; J. Schäfer et al., Plasma Process. Polym. 6 (2009), S519, J. Schäfer et al., Eur. Phys. J. D 54 (2009), 211 ; Review-Artikel: C. Tendero et al., Spectrochimica Acta Part B 61 (2006) 2; L. Bärdos et al., Thin Solid Films 518 (2010), 6705.; Patentschriften: US 006525481 B1; EP 2209354 A2 ; WO 2008/074604 A1 ; WO 2006/092614 A2 ; WO 2009/073292 A1 ; WO 2009/037331 A1 ; WO 2009/031886 A2; WO2005/0766673; WO2009/036579).

Die technischen Lösungen der Patentschriften: DE 12059831 B4, DE 102007030915A1, DE 101 16502A1 , US 2009/01888626A1 , EP 1375851A1 , DE 19534950A1 , WO 98/35379) haben folgende Nachteile:

### (a) Lage der Elektroden im Bezug zur Geometrie der Medienzufuhr:

Im Gegensatz zur erfindungsgemäßen Anordnung werden die Elektroden zumeist entweder axial und zentrisch (Spitzen, Nadel etc.) oder radial (z. B. Ringelektroden) ausgeführt. Im ersten Fall führt die Umströmung der Elektroden durch das Medium zu einer nachteiligen Flussdynamik (Turbulenzen). Im zweiten Fall entstehen axiale Gradienten der elektrischen Feldstärke, die eine langgestreckte axiale Quellengeometrie verhindern.

### (b) Isolierung der Elektroden:

Herkömmliche Plasmajets weisen oftmals metallische Elektrodenoberflächen auf, die sich in direktem Kontakt mit Plasma befinden. Für eine Oberflächenbehandlung erweist sich diese Konfiguration als nachteilig, da bedingt durch die Elektrodenerosion Metall auf die zu behandelnde Oberfläche transportiert wird und somit zu ungewünschten Kontaminationen führt. Zudem führt erhöhte Erosion zu erhöhtem Verschleiß und verkürzten Wartungsintervallen von Plasmaquellen. Das Einbetten der Elektroden in einem elektrisch isolierenden, robusten Material erweist sich bezüglich der genannten Erosion als vorteilhaft. Allerdings wird bei der herkömmlichen, flachen Geometrie (planare bzw. koplanare Entladungen) mindestens die Hälfte der Energie des elektrischen Feldes im Dielektrikum absorbiert und eine Wärmebelastung der Plasmaquelle muss dort aktiv kompensiert werden.

### (c) Anzahl der Elektroden:

Bekannte Elektroden-Arrays dienen zumeist der flächigen oder räumlichen Hochskalierung der gesamten Entladungsanordnung. Es wird außer Acht gelassen, dass ein Optimum der Elektrodenanzahl bei der räumlichen Strukturierung des elektrischen Feldes insbesondere in der azimutalen Richtung im Gasraum gefunden werden kann.

Die physikalische Auswirkung dieser Aspekte beteiligt sich synergetisch an der nachteiligen, durch die Filamentierung des Plasmas entstehenden, räumlichen Inhomogenität und an der Entstehung der lokalen plasmaphysikalischen Instabilitäten. Dies spielt eine entscheidende Rolle für die Prozesskontrolle, wenn ein Medium in das Plasma eingeführt wird.
In herkömmlichen Plasmaquellen, die das Plasma unter Atmosphärendruckbedingungen in Form eines Jets erzeugen, wird das Medium (beispielsweise ein Präkursor) direkt dem Prozessgas (nachfolgend als Quellengas bezeichnet) beigemischt oder es strömt durch ein filamentiertes, primäres Plasma. In diesen Fällen sind die reaktiven Prozesse im Medium orts- und zeitabhängig und dementsprechend heterogen. Als Folge der Heterogenität ist eine geringe Effizienz des Prozesses im gesamten Strömungsquerschnitt des Mediums zu beobachten. Das Defizit wird in der Praxis durch eine stochastische Homogenisierung des primären Plasmas oder durch die Abnahme von Reaktionsprodukten aus dem Medium in der unmittelbaren Nähe des primären Plasmas nur partiell kompensiert. Als Lösungsansätze werden entweder erhöhte Leistungen oder erhöhte Strömungsgeschwindigkeiten eingestellt.

Der auf der Leistungserhöhung basierte Lösungsansatz führt dazu, dass die Anwendung der Quelle eine erhöhte Temperaturbelastung bewirkt oder dass sie nur in einem sehr eingeschränkten Bereich ablaufen kann. Am Beispiel der Beschichtung von thermisch labilen Oberflächen kann gezeigt werden, dass eine länger andauernde Einwirkung von herkömmlichen Plasmaquellen die Oberfläche degradiert und eine kurzzeitige Einwirkung eine starke Abhängigkeit der Beschichtungsqualität vom Abstand zwischen Quelle und Oberfläche verursacht. Gleichzeitig stellt eine homogenisierende Leistungserhöhung eine weitere Einschränkung für die niederenergetischen Prozesse im Plasma dar, wie z.B. eine selektive Modifikation größerer Präkursor-Moleküle, die in einer hochenergetischen Entladung zum großem Teil zerstört werden können.

Der auf der Durchflusserhöhung basierte Lösungsweg der Homogenisierung führt dazu, dass das Plasma in solchen Quellen zum jetartigen Austritt aus der Düse geführt wird. Dieser Jet-Austritt verursacht eine hochturbulente Vermischung des Plasmas mit der umgebenden Atmosphäre und wird dadurch empfindlich gegenüber den Umgebungsbedingungen. Darüber hinaus werden Prozesse mit längerer Zeitskala im Plasma (z.B. Stoßprozesse mit den Metastabilen) ebenfalls eingeschränkt. Diese Effekte limitieren die erreichbare Qualität der Beschichtung und Oberflächenmodifikation und dadurch werden auch ihre Anwendungsbereiche eingeschränkt.

### Aufgabe der Erfindung

Der Erfindung liegt die Aufgabe zugrunde, die genannten prinzipiellen Nachteile der im Stand der Technik beschriebenen Lösungen zu beseitigen. Es handelt sich vor allem um eine neuartige Kompensation der Filamentierung des Plasmas unter Atmosphärendruck, in dessen Folge starke Gradienten der Plasmaparameter und Gastemperatur sowohl auch Turbulenzen des Gasflusses entstehen. Der Lösungsweg soll zum Erreichen einer symmetrischen homogenisierten Entladungsstruktur führen, die in der Wechselwirkung mit dem zu behandelndem Medium im Querschnitt des reaktiven Plasma-Kanals hoch effizient ist und die auch unter den laminaren Flussraten und unter den kleinen gekoppelten Energieflüssen zu einer hohen Reproduzierbarkeit der anwendungsrelevanten Prozesse führt.

### Lösung der Aufgabe

Die Aufgabe wurde durch die Konstruktion einer speziellen als Plasma-Intractor (PI) bezeichneten Plasmaquelle zur Erzeugung eines kalten, homogenen Plasmas unter Atmosphärendruckbedingungen gemäß den Merkmalen der Schutzansprüche gelöst.

### Darstellung der Erfindung

Die erfindungsgemäße Vorrichtung zur Anregung und Steuerung reaktiver Prozesse in strömenden Medien ist eine als Plasma-Intractor (PI) bezeichnete Plasmaquelle.

Die Funktionsweise der Plasmaquelle PI basiert generell auf dem Prinzip einer koplanaren **d**ielektrisch **b**ehinderten **E**ntladung (DBE) in einer dielektrischen, vorzugsweise keramischen Düse - Formkörper (1). Die DBE entsteht in einem durch die Düse im Hohlraum (7) strömenden Gas (Quellengas, 6) unter Atmosphärenbedingungen. Wird durch eine vorzugsweise konzentrisch in der Düse angeordnete Kapillare (4) oder Rohr (der Durchmesser der Anordnung kann in Größenordnung von mm bis cm skaliert werden) ein geeignetes Medium (5) dosiert in das durch die DBE generierte Plasma (nachfolgend als primäres Plasma bezeichnet, 10) eingemischt, werden reaktive Prozesse im Medium (5) angeregt. Im Falle eines gasförmigen Mediums (5) führt diese Anregung zur Zündung einer sekundären Entladung (11a) im Medium (5), die als ein Effluent (11b) aus der Kapillare (4) herausströmt. Der Prozess der Kopplung des primären homogenen Plasmas in das strömende Medium (5) und die resultierende Erzeugung des sekundären Plasmas (10) im Medium ist der Kernmechanismus der Plasmaquelle PI und wird als "Plasma-Intraktion" bezeichnet.

Eine hohe Stabilität, Homogenität und Effizienz der Plasma-Intraktion wird durch eine neue Konfiguration des elektrischen Feldes im Hohlraum erreicht. Im Gegensatz zu den herkömmlichen Plasmaquellen, sind die Elektroden (2) equidistant, exzentrisch und länglich im Formkörper (1) angeordnet, so dass die größte Feldstärke des Anregungsfeldes bevorzugt im Hohlraum (7) lokalisiert ist. Diese Eigenschaft führt zu enormer Erhöhung der Effizienz des PI. Darüber hinaus sorgt die symmetrische Anordnung der Elektroden (2) für eine optimale Verteilung der Feldstärke im Querschnitt des Hohlraumes (7), wobei eine längliche mit der Achse des Hohlraumes (7) parallele Lagerung der Elektroden (2) gradienten-freie Bedingungen in der axialen Richtung über größeren Längen erzeugt. Die Anzahl der Elektroden ist geradzahlig, die optimale Anzahl der Elektroden ist dem Durchmesser des Hohlraumes (7) anzupassen. Bei Düsen mit Innendurchmesser größer gleich 4 mm ist die Mindestanzahl der Elektroden für den gewünschten Effekt gleich 6.

Die Reaktivität des sekundären Plasmas (11) im Effluenten (11 b) wird durch den aus dem primären Plasma (10) freigesetzten Energieeintrag und durch die Zusammensetzung des strömenden Mediums maßgeblich beeinflusst. Die Einkopplung kann durch die Position der Kapillare in der Düse und die Strömungsgeschwindigkeit des Mediums (5) gesteuert werden. Das reaktive Plasma-Gas-Gemisch kann für diverse Anwendungsziele (z.B. für die Abscheidung von dünnen Schichten der Größenordnung von nm bis µm, Oberflächenfunktionalisierung von Kunststoffen, Feinreinigung oder auch für das Ätzen) eingesetzt werden.

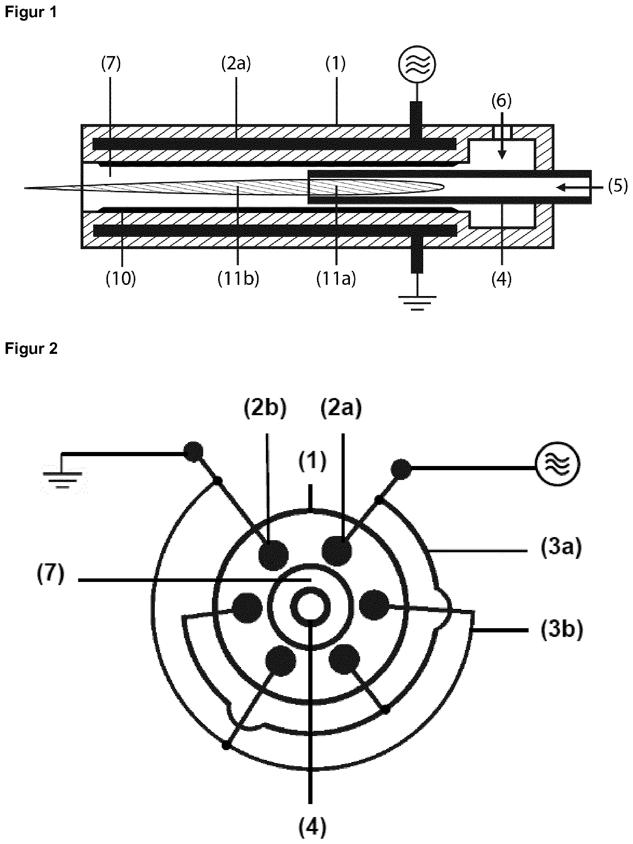

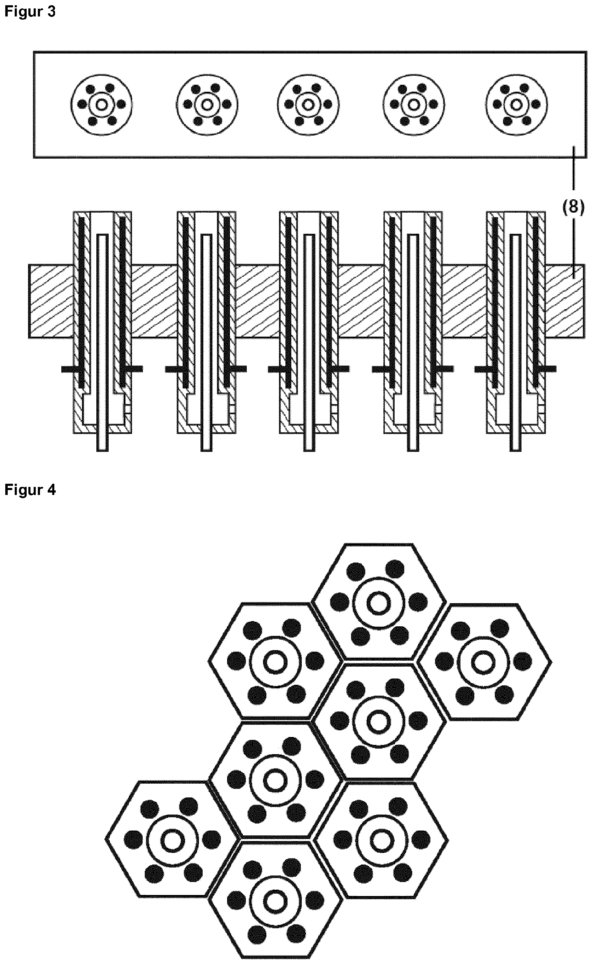

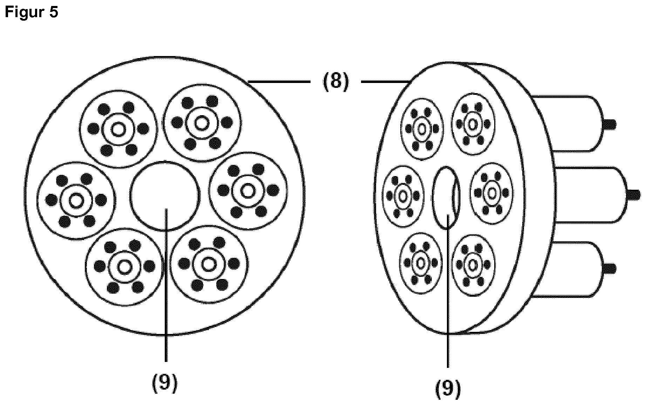

Zum Anregen des primären Plasmas (10) werden die Elektroden im Formkörper paarweise vorgespannt (s. Figur 2). Eine Hälfte der Elektroden wird geerdet, die andere wird mit einer Wechselspannung belegt. Die Polarität der benachbarten Elektroden in jeder Phase der Spannung ist gegensätzlich. Die möglichen Konfigurationen der Elektroden und unterschiedliche Formen und Anordnungen der Düsen werden anhand entsprechender Zeichnungen von Ausführungsbeispielen erläutert (Figuren 3-5). Im azimutalen Ausschnitt der Düse weist die Konfiguration eine Ähnlichkeit zu einer koplanaren Plasmaquelle. Die ebenen koplanaren Plasmaquellen benötigen allerdings größere Elektrodenpotentiale im Betrieb, dadurch wird auch ein großer Teil der Energie im Dielektrikum absorbiert und die Quelle muss typischerweise aktiv gekühlt werden. Im Gegensatz dazu begünstigt die symmetrisch exzentrische "zusammengerollte" Elektrodenkonfiguration des PI die Ankopplung des elektrischen Feldes im Innenbereich der Düse mit wesentlich größerer Effizienz. Die Erzeugung des primären Plasmas ist dadurch nicht mehr mit Temperaturbelastung verbunden. Sie erfolgt bei kleinerer Spannung als im Falle einer vergleichbaren koplanaren Plasmaquelle (US Patent Appl. No. 2004/0194223 A1 oder US 4652318 A) und die Quelle selbst benötigt keine aktive Kühlung. Darüber hinaus erhält sich der PI seine Elektrodenfreiheit, die für koplanare Plasmaquellen typisch ist. Die Vermeidung des Kontaktes von metallischen Oberflächen mit dem Plasma (Elektrodenfreiheit) verringert eine metallische Kontamination des Plasmas und der zu bearbeitenden Medien (5) durch von der Elektrodenoberfläche erodiertes Material und reduziert darüber hinaus auch den mit der Wartung des PI verbundenen Aufwand.

Im PI werden Eigenschaften von koplanaren DBE und Plasmajets vorteilhaft kombiniert, um die Homogenität des Plasmas in der Länge und die Stabilität des Plasmas im Querschnitt der Düse in besonderem Maße positiv zu beeinflussen. Durch die Kombination der symmetrischen räumlichen Struktur des Plasmas auf der Innenwand des Hohlraumes (7) und des laminaren axialen Gasfluss (6) entsteht ein Synergie-Effekt, der zur Stabilisierung und Homogenisierung des Plasmas beiträgt. In diesem Fall sind die Strömungslinien des Gases senkrecht zu den elektrischen Feldlinien orientiert. Dadurch werden die Konzentrationen der langlebenden oder geladene Plasmaspezies axial gleichmäßig verteilt. Überraschenderweise wird die Entladungsstruktur auch in denjenigen Quellengasen visuell axial homogen, die unter vergleichbaren Bedingungen in anderen herkömmlichen Plasmaquellen eine charakteristische Filamentierung aufweisen.

Der homogenisierende Synergie-Effekt im PI führt dazu, dass die effektiven Grenzen der Entladungsstruktur erweitert werden, und dass ein radialer Transport der Ladungsträger in der Düse von der Düsenwand (10) bis zur Achse der Düse erfolgen kann. Führt man durch eine zentrale Kapillare (4) ein Medium (5) in die Düse (1) mittig ein, wird bei günstigen Bedingungen (Durchfluss und chemische Zusammensetzung des Mediums) im Medium in der Kapillare ein sekundäres Plasma erzeugt. Im gasförmigen Medium ist das sekundäre Plasma (11) an die Strömung des Mediums (5) gekoppelt ("intraktiert") und die Reaktivität des Mediums kann erstaunlicherweise weiter weg aus der Düse übertragen werden. Für das Ausmaß der Plasma-Intraktion sind dann die Position der Kapillare in der Düse und die Strömungsparameter des Mediums entscheidend.

Der Effekt der Plasma-Intraktion weist mehrere anwendungsrelevante Vorteile auf. Die separat einstellbare Strömungsgeschwindigkeit des Mediums (5) ermöglicht eine effiziente Regelung des Transports von Reaktionsprodukten ohne jegliche Veränderung der Eigenschaften des primären Plasmas (10). Dies ermöglicht ein stabiles Verfahren mit geringerem Einfluss des Abstandes von der Düse zum Target (Position der Abnahme von Reaktionsprodukten). Eine homogenisierte und zylindrisch symmetrische Wechselwirkung zwischen primärem Plasma (10) und dem Medium (5) führt zu einer hohen Effizienz des Prozesses. Am Beispiel der Schichtabscheidung von SiOₓ-Schichten mithilfe eines PI-Prototyps wurde dadurch eine unerwartet hohe Ausbeute festgestellt, was in der Praxis eine Zeit- und Kostenersparnis darstellt.

Ein weiterer wesentlicher Vorteil der Vorrichtung besteht in der geringen Temperatur des sekundären Plasmas. Dadurch, dass das sekundäre Plasma im Medium nicht direkt durch die primäre elektrische Feldstärke erzeugt wird, sondern durch die hohe Dichte an bereits erzeugten ionisationsfähigen Spezies des primären Plasmas, ist die mittlere kinetische Temperatur des sekundären Plasmas maßgeblich durch die Neutraltemperatur des Mediums bestimmt. Dies ermöglicht auch Prozesse mit thermolabilen oder biologischen Materialien.

### Ausführungsbeispiele

Die Erfindung soll nachfolgend anhand einiger Beispiele näher erläutert werden, ohne die Erfindung auf diese Beispiele zu beschränken. Die Figuren 1, 2, 3, 4 und 5 zeigen Beispiele für die die erfindungsgemäße Vorrichtung.

Für die nachfolgenden Zeichnungen werden folgende Bezugszeichen verwendet:
- (1): Formkörper (z. B. Keramikdüse)
- (2): Elektroden (2a: Hochspannungselektrode, 2b: geerdete Elektrode)
- (3): Verbindungsleitung (3a: der Hochspannungselektroden, 3b: der geerdeten Elektroden)
- (4): Kapillare (z.B. Quarzkapillare)
- (5): Zufuhr des Mediums
- (6): Gasfluss (Prozessgas bzw. "Quellengas")
- (7): Hohlraum
- (8): Grundplatte
- (9): Kanal für zusätzliche Mediumzufuhr
- (10): primäres Plasma
- (11): sekundäres Plasma, 11 a - Erzeugung, 11 b - Effluent

### Beispiel 1: Bevorzugte Ausführungsform eines einzelnen PI

Figur 1 zeigt die schematische Darstellung der Düsenanordnung eines einzelnen PI im Längsschnitt und Fig.2 dieselbe Anordnung in der Frontansicht.

Herzstück des PI ist ein Formkörper (1) mit 6 Ausnehmungen für Elektroden (2) und einem axialen zylindrischen Hohlraum (7). Der Hohlraum (7) dient dabei als Gasraum für die Generierung des primären Plasmas (10). In den weiteren sechs Ausnehmungen, die äquidistant zueinander um den Hohlraum herum angeordnet sind, sind die Elektroden (2a und 2b) eingebettet. Die koaxial eingeführte Keramik-Kapillare (4) dient der Zufuhr eines Mediums (5), beispielsweise eines Aerosols aus einem Trägergas und einem Präkursor im Falle einer Anwendung für Beschichtungen. Das Prozessgas (6) strömt durch eine entsprechende Öffnung am hinteren Ende der Düse in den Hohlraum (7). Primäres Plasma (10) ist an der Wand des Hohlraumes lokalisiert, sekundäres Plasma (11) entsteht im axialen Bereich der Kapillare (4) und hat eine weitführende Wirkung nach dem Austritt aus der Düse.

Figur 2 demonstriert die symmetrische Anordnung von Hochspannungselektroden (2a) und geerdeten Elektroden (2b), die außen über entsprechende Verbindungsleitungen (3a und 3b) für die Hochspannungselektroden und für die geerdeten Elektroden kontaktiert sind. Diese bevorzugte Ausführungsform der Erfindung kann unter folgenden Bedingungen effektiv als PI genutzt werden: Arbeitsgas: Argon, Betriebsfrequenz von wenigen kHz bis MHz, Betriebsspannung bei 10 kHz von 5 bis 15 kVpp, entsprechende Leistung bereits von 1 bis 10 W pro Düse (Länge der Düse 2 cm).

Die folgenden Figuren zeigen mögliche Kombinationen mehrerer einzelner PI in einem Array (Figur 3), in einer Matrix (Figur 4) sowie in einer konzentrischen Konfiguration (Figur 5). In diesen Fällen werden die Düsen jeweils auf einer entsprechend gestalteten Grundplatte (8) montiert. Im Fall der konzentrischen Anordnung (Figur 5) kann der zentrale Kanal (9) für eine weitere Medienzufuhr genutzt werden.

Beispiel 2 zeigt eine schematische Darstellung einer zeilenförmigen Anordnung von 5 PI.

Beispiel 3 zeigt eine schematische Darstellung einer matrixförmigen Anordnung mehrerer PI.

Beispiel 4 zeigt die schematische Darstellung einer konzentrischen Anordnung von sechs PI

## Patentansprüche

1. Vorrichtung zur Erzeugung eines kalten homogenen Plasmas unter Atmosphärendruckbedingungen, umfassend mindestens 6 länglich ausgeführte Elektroden (2) und einen Formkörper (1) aus Isolatormaterial, wobei der Formkörper (1) mit einem länglichen zylindrischen Hohlraum (7) und mit weiteren mit dem Hohlraum parallel geführten, symmetrisch zur Achse des Hohlraumes und äquidistant zueinander angeordneten Ausnehmungen vorgesehen ist und die Elektroden (2) in Ausnehmungen des Formkörpers (1) eingebettet sind und diese so mit einer AC-Hochspannungsversorgung verbunden sind, dass die Polaritäten jeweils benachbarter Elektroden in jeder Phase der Spannungsperiode gegensätzlich sind, **dadurch gekennzeichnet dass** in dem Hohlraum (7) des Formkörpers (1) eine in der axialen Lage einstellbare Kapillare (4) aus Isolatormaterial angeordnet ist.

2. Vorrichtung nach Anspruch 1, **dadurch gekennzeichnet, dass** in dem Hohlraum (7) des Formkörpers (1) ein zu behandelndes Werkstück eingebracht und durchgeführt werden kann.

3. Vorrichtung nach Anspruch 2 **dadurch gekennzeichnet, dass** das Werkstück ein Stab, ein Draht, ein Geflecht, ein Seil, ein Rohr oder eine Faser sein kann.

4. Vorrichtung nach Anspruch 1, **dadurch gekennzeichnet, dass** über die Kapillare Gase, Dampfe oder Flüssigkeiten in das Plasma eingebracht werden können.

5. Verfahren zur Erzeugung eines homogenen Plasmas unter Atmosphärendruckbedingungen nach dem Prinzip der dielektrisch behinderten Entladung (DBE), **dadurch gekennzeichnet, dass** nach dem Prinzip der DBE ein primäres Plasma in dem Hohlraum (7) einer Vorrichtung gemäß Anspruch 1 bis 4 erzeugt wird und mittels einer dielektrischen Kapillare (4) in dem Hohlraum (7), durch die gasförmiges Medium strömt, ein sekundäres Plasma separat erzeugt wird.

6. Verfahren nach Anspruch 5, **dadurch gekennzeichnet, dass** die Reaktivität des sekundären Plasmas durch die Energie aus dem primären Plasma oder durch die Zusammensetzung des strömenden Mediums in der Kapillare (4) oder der Position der Kapillare (4) in dem Hohlraum (7) gesteuert wird.

7. Verwendung der Vorrichtung gemäß den Ansprüchen 1 bis 4 zur
a) externen Oberflächenmodifizierung oder
b) Erzeugung aktiver chemischer Verbindungen oder reaktiver Teilchen zur Modifizierung von Oberflächen oder zur
c) internen reaktiven Modifizierung von heterogenen Substenzen in einem strömenden Medium oder zur
d) plasmagestützten Synthese komplexer Produkte ausgewählt aus der Gruppe umfassend Cluster, Nanopartikel und chemische Verbindungen aus einem strömenden Medium ausgewählt aus der Gruppe umfassend reaktive Gasmischungen, Rauche und Aerosole, oder zur
e) Umwandlung oder Dekontamination eines komplexen Mediums oder zum
f) Materialabtrag von einer Oberfläche.

8. Array oder Matrix, die mehrere Vorrichtungen gemäß den Ansprüchen 1 bis 4 enthält.

## Claims

1. A device for producing a cold, homogeneous plasma under atmospheric pressure conditions, comprising at least 6 electrodes (2) of elongated design and a moulded body (1) made of insulating material, wherein the moulded body (1) is provided with an elongated cylindrical cavity (7) and with further recesses guided parallel to the cavity and arranged symmetrically to the axis of the cavity and equidistant from one another and the electrodes (2) are embedded in recesses of the moulded body (1) and said electrodes are connected to an AC high voltage supply such that the polarities of the respective adjacent electrodes are opposite in every phase of the voltage period, **characterized in that** a capillary (4) which is adjustable in axial position and which is made of insulating material is arranged in the cavity (7) of the moulded body (1).

2. The device according to Claim 1, **characterized in that** a workpiece to be treated may be introduced and processed in the cavity (7) of the moulded body (1).

3. The device according to Claim 2, **characterized in that** the workpiece may be a rod, a wire, a braid, a cable, a tube or a fibre.

4. The device according to Claim 1, **characterized in that** gases, vapours or liquids may be introduced via the capillary into the plasma.

5. A method for producing a homogeneous plasma under atmospheric pressure conditions according to the principle of dielectrically hindered discharge (DHD), **characterized in that** according to the principle of DHD a primary plasma is produced in the cavity (7) of a device according to Claim 1 to 4 and a secondary plasma is separately produced by means of a dielectric capillary (4) in the cavity (7), a gaseous medium flowing through said capillary.

6. The method according to Claim 5, **characterized in that** the reactivity of the secondary plasma is controlled by the energy from the primary plasma or by the composition of the flowing medium in the capillary (4) or the position of the capillary (4) in the cavity (7).

7. A use of the device according to Claims 1 to 4 for
a) external surface modification or
b) producing active chemical compounds or reactive particles for modifying surfaces or for
c) internal reactive modification of heterogeneous substances in a flowing medium or for
d) plasma-assisted synthesis of complex products selected from the group comprising clusters, nanoparticles and chemical compounds from a flowing medium selected from the group comprising reactive gas mixtures, smoke and aerosols, or for
e) converting or decontaminating a complex medium or for
f) removal of material from a surface.

8. An array or matrix which contains a plurality of devices according to Claims 1 to 4.

## Revendications

1. Dispositif de production d'un plasma froid homogène dans des conditions de pression atmosphérique, comprenant au moins 6 électrodes (2) conçus de forme allongée et un corps moulé (1) constitué d'un matériau isolant, le corps moulé (1) étant prévu avec une cavité (7) cylindrique allongée et avec d'autres évidements parallèles à la cavité et agencés symétriquement à l'axe de la cavité et équidistants les uns des autres, et les électrodes (2) étant incorporées dans des évidements du corps moulé (1) et celles-ci étant connectées à une alimentation haute tension CA de telle sorte que les polarités d'électrodes voisines respectives sont opposées dans chaque phase de la période de tension, **caractérisé en ce qu'**un capillaire (4) constitué d'un matériau isolant pouvant être réglé dans la position axiale est disposé dans la cavité (7) du corps moulé (1).

2. Dispositif selon la revendication 1, **caractérisé en ce qu'**une pièce à traiter est mise en place dans la cavité (7) du corps moulé (1) et peut être réalisée.

3. Dispositif selon la revendication 2, **caractérisé en ce que** la pièce peut être une barre, un fil métallique, un treillis, un câble, un tube ou une fibre.

4. Dispositif selon la revendication 1, **caractérisé en ce que** des gaz, des vapeurs ou des liquides peuvent être mis en place dans le plasma par le biais du capillaire.

5. Procédé de production d'un plasma homogène dans des conditions de pression atmosphérique selon le principe de la décharge à barrière diélectrique (DBD), **caractérisé en ce que**, selon le principe de la DBD, un plasma primaire est produit dans la cavité (7) d'un dispositif selon les revendications 1 à 4 et **en ce qu'**un plasma secondaire est produit séparément au moyen d'un capillaire (4) diélectrique dans la cavité (7) dans lequel s'écoule un milieu gazeux.

6. Procédé selon la revendication 5, **caractérisé en ce que** la réactivité du plasma secondaire est commandée par l'énergie en provenance du plasma primaire ou par la composition du milieu s'écoulant dans le capillaire (4) ou de la position du capillaire (4) dans la cavité (7).

7. Utilisation du dispositif selon les revendications 1 à 4 pour
a) la modification de surface externe ou pour
b) la production de composés chimiques actifs ou de particules réactives pour la modification de surfaces ou pour
c) la modification réactive interne de substances hétérogènes dans un milieu en écoulement ou pour
d) la synthèse, assistée par plasma, de produits complexes sélectionnés dans le groupe comprenant des clusters, des nanoparticules et des composés chimiques en provenance d'un milieu en écoulement sélectionné dans le groupe comprenant des mélanges gazeux réactifs, des fumées ou des aérosols, ou pour
e) la transformation ou la décontamination d'un milieu complexe ou pour
f) l'enlèvement de matière à partir d'une surface.

8. Array ou matrice qui contient plusieurs dispositifs selon les revendications 1 à 4.
